# EUROPÄISCHE PATENTSCHRIFT

(11) **EP 1 675 816 B1**
(45) Veröffentlichungstag und Bekanntmachung des Hinweises auf die Patenterteilung: **20.06.2007**
(21) Anmeldenummer: 04765773.9
(22) Anmeldetag: 02.10.2004
(51) Int. Cl.: C07C 213/10, C07C 215/12

(54) **VERFAHREN ZUR ABTRENNUNG VON TRIETHANOLAMIN AUS EINEM DURCH DIE UMSETZUNG VON AMMONIAK MIT ETHYLENOXID ERHALTENEN STOFFGEMISCH**
METHOD FOR SEPARATING TRIETHANOLAMIN FROM A MIXTURE OBTAINABLE BY AMMONIA AND ETHYLENE OXIDE REACTION
PROCEDE DE SEPARATION DE TRIETHANOLAMINE D'UN MELANGE DE SUBSTANCES OBTENU PAR REACTION D'AMMONIAQUE AVEC DE L'OXYDE D'ETHYLENE

(30) Priorität: 08.10.2003 DE 10346779
(43) Veröffentlichungstag der Anmeldung: 05.07.2006
(73) Patentinhaber: BASF Aktiengesellschaft, 67056 Ludwigshafen (DE)
(72) Erfinder: REIF, Wolfgang, 67227 Frankenthal (DE); HAMMER, Hans, 68219 Mannheim (DE); RUIDER, Günther, 67157 Wachenheim (DE); MEIER, Anton, 67134 Birkenheide (DE); BUSKENS, Philipp, B-2320 Hoogstraten (BE); FRAUENKRON, Matthias, 67251 Freinsheim (DE)
(86) Internationale Anmeldenummer: PCT/EP2004/011022
(87) Internationale Veröffentlichungsnummer: WO 2005/035481

(56) Entgegenhaltungen:
- US-A- 3 453 183
- US-A- 3 742 059
- US-A- 4 673 762
- US-A1- 2001 031 897
- US-A1- 2004 127 748
- PATENT ABSTRACTS OF JAPAN Bd. 011, Nr. 193 (C-430), 20. Juni 1987 (1987-06-20) & JP 62 019558 A (MITSUI TOATSU CHEM INC), 28. Januar 1987 (1987-01-28) in der Anmeldung erwähnt

## Beschreibung

Die Erfindung betrifft ein Verfahren zur kontinuierlichen, destillativen Abtrennung von Triethanolamin (TEA) aus einem durch die Umsetzung von Ammoniak mit Ethylenoxid in flüssiger Phase unter Druck und bei erhöhter Temperatur erhaltenen Stoffgemisch aus Mono-, Di- und Triethanolamin sowie Ammoniak, Wasser und Ethanolamin-Ether.

Es ist allgemein bekannt, dass ein nach einer fraktionierenden Destillation eines TEA-Rohprodukts, das durch Umsetzung von wässrigem Ammoniak mit Ethylenoxid und Abdestillieren von Monoethanolamin (MEA) und Diethanolamin (DEA) gewonnen wurde, erhaltenes und zunächst farbloses, reines TEA (Farbzahl: ca. 0 bis 20 APHA nach DIN-ISO 6271 (= Hazen)), sich nach einer Lagerzeit von ca. 4 bis 6 Wochen, auch im geschlossenen Gebinde und unter Lichtausschluss, allmählich leicht rosa und schließlich, besonders leicht beim Stehen am Licht, gelb bis braun verfärben kann. Dieser Effekt wird durch Einwirkung von höheren Temperaturen beschleunigt. (Siehe z.B.: G.G. Smirnova et al., J. of Applied Chemistry of the USSR 61, S. 1508-9 (1988), und Chemical & Engineering News 1996, Sept. 16, Seite 42, mittlere Spalte).

Gemäß Chemical & Engineering News 1996, Sept. 16, Seite 42, zersetzt sich bei erhöhter Temperatur ein Mol TEA in ein Mol Ethanolamin und zwei Mol Acetaldehyd. Acetaldehyd kondensiert zu Crotonaldehyd, der wiederum mit Ethanolamin eine Schiff'sche Base bildet. Diese ungesättigte Schiff'sche Base führt unter 1,4-Polymerisation zu farbigen Produkten im TEA.

Zur Beurteilung der Farbqualität vom reinem TEA hat sich neben den zeitaufwendigen Lagerversuchen, bei denen die APHA-Farbzahl (nach DIN-ISO 6271) des TEAs in Abhängigkeit von der Lagerzeit gemessen wird, der sogenannte Säureneutralisationstest bewährt.

Dieser Säureneutralisationstest erlaubt die Beurteilung der farblichen Lagerstabilität von frisch hergestelltem TEA innerhalb weniger Minuten.

Der Test wird in den japanischen Dokumenten JP-A-62 019 558 (Derwent Abstract Nr. 87-067647/10) und JP-A-62 005 939 (Derwent Abstract Nr. 87-047397/07) beschrieben, wonach das TEA mit Essigsäure, Zitronensäure, Schwefelsäure, Salzsäure oder Phosphorsäure behandelt (neutralisiert) wird und danach die Extinktion der Absorptionsbanden bei 420 nm und 530 nm gemessen wird. Tritt während der Durchführung des Tests keine augenscheinliche Rosaverfärbung des TEAs auf und bleiben die gemessenen Werte für die Extinktion genügend klein, so ist das TEA farblich lagerstabil, bleibt also über einen Zeitraum von mehreren Monaten farblos.

In der Literatur sind verschiedene Verfahren zur Herstellung von reinem und farblosem bis wenig gefärbtem TEA beschrieben.

EP-A-4015 beschreibt, dass Ethanolamine mit geringerer Verfärbung durch Zusatz von phosphoriger oder unterphosphoriger Säure während der Herstellung der Ethanolamine und/oder der destillativen Aufarbeitung erhalten werden.

EP-A-36 152 und EP-A-4015 erläutern den Einfluss der in Verfahren zur Herstellung von Alkanolaminen eingesetzten Werkstoffe auf die farbliche Qualität der Verfahrensprodukte und empfehlen nickelfreie bzw. nickelarme Stähle.

US-A-3 819 710 offenbart ein Verfahren zur Verbesserung der Farbqualität von Ethanolaminen durch Hydrierung der rohen Ethanolamine in Gegenwart ausgewählter Katalysatoren. Das Verfahren ist jedoch technisch aufwendig und führt nicht zu einer TEA-Ware, die über mehrere Monate farblos bleibt.

US-A-3 207 790 beschreibt ein Verfahren zur Verbesserung der Farbqualität von Alkanolaminen durch Zugabe eines Borhydrids eines Alkalimetalls.

US-A-3 742 059 und DE-A-22 25 015 beschreiben die Verbesserung der Farbqualität von Alkanolaminen durch den Zusatz eines Alkanolaminesters der Borsäure bzw. Alkali-/Erdalkalimetallboraten.

Die Anwesenheit eines Hilfsstoffes zur Stabilisierung von TEA ist jedoch in vielen wichtigen Anwendungsbereichen des TEAs unerwünscht.

Die nachträgliche Zugabe kleiner Mengen Ethylenoxid zu frisch destilliertem TEA führt nach US-A-4 673 762 ebenfalls zu einer Entfärbung und Farbstabilisierung. Die Methode erscheint jedoch aus toxikologischen Gründen bedenklich.

GB-A-1 062 730 beschreibt ein Verfahren zur Reinigung von Ethanolaminen durch Reindestillation in Gegenwart von Silikaten oder Aluminaten.

JP-A-62 019 558 (Derwent Abstract Nr. 87-067647/10) berichtet über die Herstellung von qualitativ gutem TEA durch Behandlung von rohem TEA mit anorganischen Oxiden bei 170 bis 250°C und anschließender Destillation in Abwesenheit von Sauerstoff.

Ähnliche Ergebnisse werden gemäß JP-A-62 005 939 (Derwent Abstract Nr. 87-047397/07) erzielt, wenn rohes TEA unter Luftausschluss 1 bis 10 h auf 170 bis 250°C erhitzt und dann im Vakuum destilliert wird.

SU-A-326 178 (Derwent Abstract Nr. 63384T-AE) beschreibt die Herstellung TEA mit guter Farbqualität durch schonende Umsetzung von wasserfreiem Monoethanolamin (MEA) oder Diethanolamin (DEA) oder Mischungen beider Substanzen mit Ethylenoxid bei Temperaturen kleiner 50°C.

Ähnliche Ergebnisse werden gemäß SU-A-228 693 (Chem. Abstr. 70, 77305f (1969)) und GB-A-1 092 449 erzielt, wenn Ammoniak mit Ethylenoxid bei kleiner/gleich 35°C umgesetzt und die erhaltene Ethanolamin-Mischung unter Luftausschluss destilliert wird.

Vom wirtschaftlichen Gesichtspunkt sind solche Verfahren, bei denen die Umsetzungen mit Ethylenoxid bei niedrigen Temperaturen stattfinden, wegen der langen Verweilzeiten und der damit verbundenen niedrigen Raum-Zeit-Ausbeuten unrentabel.

Es war daher Aufgabe der Erfindung, ein Verfahren zur Abtrennung von Triethanolamin aus einem durch die Umsetzung von Ammoniak mit Ethylenoxid erhaltenen Stoffgemisch zu schaffen, nach dem eine hohe Reinheit des Triethanolamins erreicht werden kann. Die Herstellung von verkaufsfähiger Triethanolamin-Reinware soll zudem besonders einfach und wirtschaftlich sein.

Zur Lösung dieser Aufgabe werden die im Kennzeichen des Anspruchs 1 enthaltenen Maßnahmen vorgeschlagen.

Weitere Maßnahmen ergeben sich aus den Unteransprüchen 2 bis 4.

Die Erfindung verwendet ein Stoffgemisch, welches wie folgt erhalten wird. Zunächst wird z.B. gemäß EP-A-673 920, durch die Umsetzung von wässrigem Ammoniak mit Ethylenoxid in flüssiger Phase unter Druck und erhöhter Temperatur in einem geeigneten Reaktor ein Ethanolamin-Gemisch, enthaltend die Hauptkomponenten Monoethanolamin (MEA), Diethanolamin (DEA) und Triethanolamin (TEA), hergestellt.

Die Reaktionstemperaturen betragen hierbei im allgemeinen 110 bis 180°C, bevorzugt 120 bis 150°C, und die Drücke 50 bis 150 bar (5 bis 15 MPa), bevorzugt 75 bis 120 bar (7,5 bis 12 MPa). Das molare Verhältnis von Ammoniak zu Ethylenoxid beträgt 1 : 1 bis 100 : 1, bevorzugt 3 : 1 bis 50 : 1, besonders bevorzugt 4 : 1 bis 15 : 1, und der Ammoniak wird als 60 bis 99,99 %ige, bevorzugt 70 bis 95 %ige, wässrige Lösung eingesetzt. Das eingesetzte Ethylenoxid kann als Gesamtmenge auf einmal oder in zwei bis zehn, bevorzugt zwei bis sechs, Teilmengen von jeweils 10 bis 70 Gew.-% (bezogen auf die Gesamtmenge) zugegeben werden.

Arbeitet man mit einem molaren Verhältnis von Ammoniak zu Ethylenoxid von mehr als 1:1. wird aus dem erhaltenen Produktgemisch anschließend in an sich bekannter Weise der überschüssige Ammoniak zusammen mit einem Teil des Wassers unter Druck und danach das restliche Wasser bei vermindertem Druck oder drucklos abdestilliert. Zurück bleibt ein im wesentlichen MEA, DEA und TEA enthaltendes Rohprodukt mit einem Wassergehalt kleiner 0,3 Gew.-%, bevorzugt kleiner 0,1 Gew.%.

Nach der sich anschließenden destillativen Abtrennung des Monoethanolamins (MEA) bei vermindertem Druck verbleibt ein Rohprodukt bestehend aus DEA, TEA und geringen Mengen an Nebenkomponenten, wie beispielsweise (2-(2-Hydroxyethoxy)ethyl)-di-(2-hydroxyethyl)-amin, (2-(2-Hydroxyethoxy)ethyl)-(2-hydroxyethyl)-amin und N,N'-Di-(2-hydroxyethyl)-piperazin.-Ein typisches Rohgemisch enthält ca. 70 Gew.-% DEA und ca. 30 Gew.-% TEA.

Die Zusammensetzung dieses Rohprodukts kann je nach dem ursprünglich eingesetzten molaren Verhältnis von Ammoniak zu Ethylenoxid schwanken.

In der Regel kann das so erhaltene Ethanolamin-Gemisch direkt einer fraktionierenden Destillation unterworfen werden, bei der nacheinander reines DEA und TEA erhalten werden. Alternativ ist jedoch eine Vorgehensweise möglich, bei der dieses, im wesentlichen DEA und TEA enthaltende Rohprodukt, das einen Wassergehalt kleiner 0,3 Gew.-%, bevorzugt kleiner 0,1 Gew.-%, und einen Ammoniakgehalt kleiner 0,1 % Gew.-%, bevorzugt kleiner 0,01 Gew.-%, aufweist, mit 0,6 bis 1,2 Mol, bevorzugt 0,8 bis 1,1 Mol Ethylenoxid pro Grammatom an Stickstoff gebundenen Wasserstoff im Rohprodukt bei Temperaturen von 110 bis 180°C, bevorzugt 120°C bis 180°C, in flüssiger Phase umgesetzt wird. Diese Umsetzung erfolgt im allgemeinen wie in der GB-A-1 453 762 beschrieben. Bevorzugt erfolgt die Umsetzung in Rohrreaktoren und mehrstufig, wobei beispielsweise in einer ersten Reaktionsstufe bei Temperaturen von vorzugsweise 125 bis 165°C 50 bis 80 Gew.-% des eingesetzten Ethylenoxids, in einer zweiten Reaktionsstufe bei Temperaturen von vorzugsweise 150 bis 180°C die restliche Menge des eingesetzten Ethylenoxids umgesetzt wird und in einer dritten Umsetzungsstufe die Reaktion bei Temperaturen von 120 bis 150°C zu Ende geführt wird.

Das so erhaltene Stoffgemisch aus MEA, DEA und TEA sowie Ethanolamin-Ether und Wasser wird nach der Erfindung in zwei Stufen destilliert. Hierfür eignen sich übliche Destillationsvorrichtungen. Solche Vorrichtungen sind einem Fachmann bekannt. Es wird bevorzugt, eine Destillationskolonne mit wenigstens einer Quer- oder Längsunterteilung, ausgeführt in Form eines Bodens, einer Trennwand, geordneter Packungen oder Füllkörper, zu verwenden. Die Kolonne wird mit einer Temperatur im Sumpf von zweckmäßig 160°C bis 210°C betrieben. Der gewählte Druck bewegt sich dabei bei Werten von 0,5 mbar bis 5 mbar. Die Kolonne wird so eingestellt, dass ein Rücklaufverhältnis von 0,05 bis 0,5, vorzugsweise von 0,1 bis 0,4 resultiert. Das aufzutrennende Stoffgemisch wird vorzugsweise in der oberen Kolonnenhälfte eingespeist.

Um ein effektives Abtrennen des Triethanolamins (TEA) zu ermöglichen, werden in einer ersten Destillationsstufe die Leichtsiederfraktion am Kolonnenkopf und die Schwersiederfraktion am Kolonnensumpf entnommen und ausgeschleust. Die verbleibende Mittelsiederfraktion mit einem Gehalt an Triethanolamin von > 98,5 Gew.-% und Diethanolamin von < 0,2 Gew.-% wird in einer zweiten Stufe destilliert.

Aufgrund der Temperaturempfindlichkeit des Stoffgemischs ist es zweckmäßig, die Kolonne mit einem Verdampfer zu betreiben, der eine geringe Wandtemperatur sowie einen kleinen Flüssigkeitsinhalt aufweist. Insgesamt hat es sich als besonders günstig erwiesen, einen Fallfilmverdampfer zu verwenden. Der Kolonnensumpf und der Verdampfersumpf werden dabei so gestaltet, dass die Verweilzeit der Schwersiederfraktion im Kolonnensumpf 1 min bis 60 min beträgt. Bei diesen Verweilzeiten wird ein Optimum aus der Abtrennung der Mittelsiederfraktion und der Vermeidung der Bildung unerwünschter Nebenprodukte erzielt.

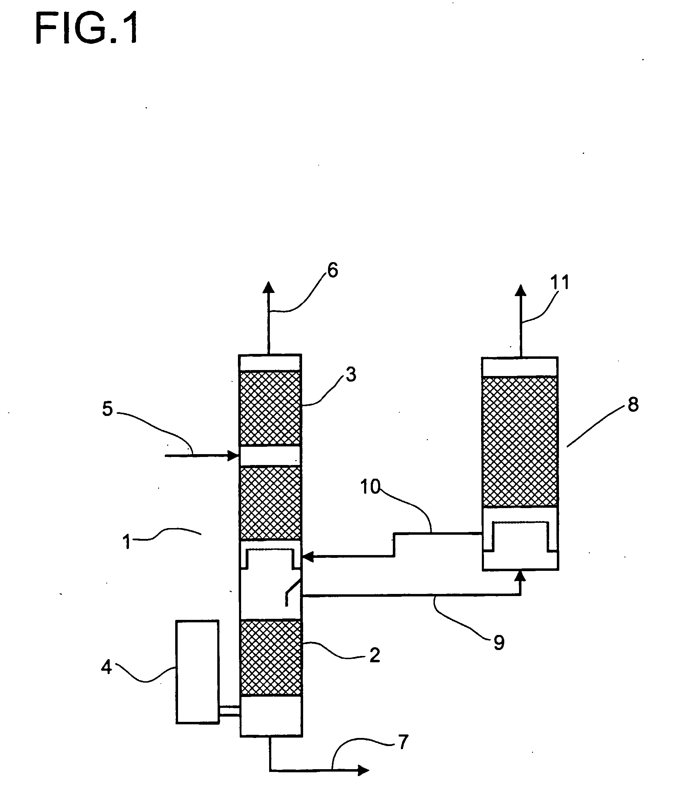

Nach einer bevorzugten Ausführungsform des erfindungsgemäßen Verfahrens erfolgt die Destillation des Stoffgemischs in zwei zusammengeschalteten Destillationskolonnen (s. Fig. 1). Dabei wird das aufzutrennende Roh-TEA-Gemisch im allgemeinen dem oberen Teil einer ersten Kolonne (1) zugeführt. Am Kopf dieser Kolonne wird eine Leichtsiederfraktion entnommen und wieder in die Ethanolamin-Aufarbeitung zurückgefahren. Im Sumpf von Kolonne (1) können bei Bedarf hochsiedende Komponenten ausgeschleust werden. Die im ersten Destillationsschritt erhaltene Mittelsiederfraktion wird an einem Seitenabzug der Kolonne (1) entnommen, in das untere Ende der zweiten Destillationsstufe - Kolonne (8) - geleitet und destilliert. Es ist vorteilhaft, auch die Kolonne (8) mit wenigstens einer Quer- oder Längsunterteilung, ausgeführt in Form eines Bodens, einer Trennwand, geordneter Packungen oder Füllkörper, zu verwenden. Die in der Kolonne (8) entstehenden Hochsieder werden wieder in den mittleren Bereich von Kolonne (1) zurückgeführt. Am Kopf der zweiten Kolonne (8) fällt reines TEA an. Das Rücklaufverhältnis in der Kolonne (8) beträgt zwischen 0,2 und 0,7. Vorzugsweise werden die erste Kolonne und die zweite Kolonne mit annähernd gleichen Temperaturprofilen betrieben.

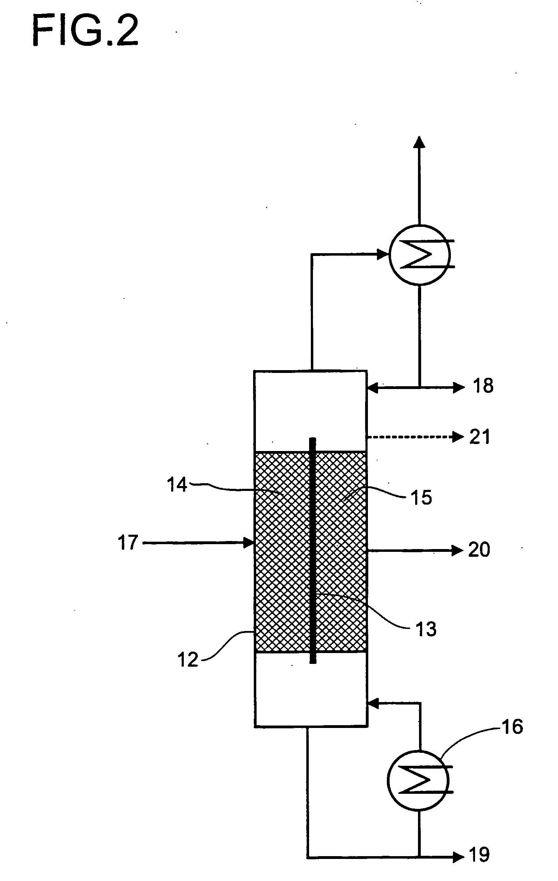

Gemäß einer besonders vorteilhaften Verfahrensvariante erfolgt die Destillation des Stoffgemischs in einer Trennwandkolonne. Eine Trennwandkolonne ist im Prinzip eine apparative Vereinfachung von zwei thermisch gekoppelten Destillationskolonnen. Sie enthält in der Regel eine senkrecht angeordnete und sich oberhalb und unterhalb der Zulaufstelle erstreckende Trennwand, welche die Kolonne in einen Zulaufteil und einen Entnahmeteil unterteilt. Zur Durchführung des erfindungsgemäßen Verfahrens kann die Trennwandkolonne als Füllkörper oder geordnete Packungen enthaltende Packungskolonne oder als Bodenkolonne ausgestaltet sein. Das Stoffgemisch wird im mittleren Bereich der Trennwand (13) in die Kolonne (12) eingeleitet (Fig. 2). Die erste Destillationsstufe wird im Zulaufteil (14) der Kolonne durchgeführt und die nach Entnahme der Leichtsiederfraktion am Kolonnenkopf und Schwersiederfraktion am Kolonnensumpf verbleibende Mittelsiederfraktion wird im Entnahmeteil (15) der Kolonne destilliert, wobei der Austrag von reinem Triethanolamin (TEA) über einen Seitenabzug (20) im mittleren Bereich der Trennwand erfolgt und die in der zweiten Destillationsstufe entstehenden Hochsieder ebenfalls im Kolonnensumpf ausgeschleust werden.
Das erfindungsgemäße Verfahren liefert Triethanolamin (TEA) mit einem Reinheitsgrad von > 99,4 % und einer APHA-Farbzahl von ≦ 30.

TEA in besonders guter Farbqualität mit Farbzahlen ≦ 20 und hoher Farbstabilität wird erhalten, wenn man, wie in EP 4015 beschrieben, eine wirksame Menge an Phosphoriger oder Unterphosphoriger Säure bzw. deren Derivate vor oder während der Ethanolamin-Synthese aus Ethylenoxid und Ammoniak bzw. im Laufe der destillativen Auftrennung des erhaltenen Ethanolamin-Gemisches zugibt. Vorzugsweise erfolgt die Zudosierung erst vor der TEA-Reindestillation. Erfolgt die Zugabe des Additivs nach der Umsetzung von Ethylenoxid und Ammoniak in der Destillation der daraus erhaltenen Ethanolamine, beträgt die zudosierte Menge zwischen 0,005 und 2 Gew.-% bezogen auf die Summe der Ethanolamine.

Die Erfindung wird nachfolgend unter Hinweis auf die Zeichnungen anhand von Ausführungsbeispielen näher erläutert.

Es zeigt
- Fig. 1: einen Anlagenaufbau in schematischer Darstellung mit
(1) einer ersten Kolonne umfassend
(2) ein Abtriebsteil und
(3) ein Verstärkungsteil,
(4) einem Fallfilmverdampfer,
(5) einem Kolonnenzulauf,
(6) einem Kopfabzug,
(7) einem Sumpfabzug sowie
(8) einer zweiten Kolonne, die über
(9) eine erste Verbindungsleitung und
(10) eine zweite Verbindungsleitung mit der ersten Kolonne zusammengeschaltet ist und
(11) einen Kopfabzug für den Austrag von reinem TEA aufweist.
und
- Fig. 2: eine Trennwandkolonne (12) mit
einer mittigen Trennwand (13), durch die
ein Zulaufteil (14) und
ein Entnahmeteil (15) gebildet wird,
einem Fallfilmverdampfer (16),
einem Kolonnenzulauf (17),
einem Kopfabzug (18),
einem Sumpfabzug (19),
einem Seitenabzug (20) für den Austrag von reinem TEA und
einem Seitenabzug (21) für die Entnahme eines DEA reichen Leichtsiederstromes.

### Beispiele

### Beispiel 1

340 kg/h eines Zulaufgemisches bestehend aus 76,4 % TEA, 22,4 % DEA, 1,1 % Ethanolamin-Ether, 0,02 % Wasser und 0,1 % Phosphorige Säure werden flüssig mit einer Zulauftemperatur von 180°C bei (5) der ersten Kolonne (1) zugeführt. Die Kolonne wird bei einem Kopfdruck von 2 mbar betrieben. Am Sumpfabzug (7) der Kolonne erfolgt die Ausschleusung von 50 kg/h eines Gemisches aus 93 % TEA und 6,9 % Ethanolamin-Ether bei einer Temperatur von 190°C. Die Verweilzeit der Flüssigkeit im Sumpf beträgt etwa 45 min. Am Kopfabzug (6) der Kolonne werden 120 kg/h eines Gemisches aus 67 % DEA, 32,4 % TEA und 0,4 % Ethanolamin-Ether ausgetragen und dem Ausgangsgemisch zugeführt. Die Temperatur dieses Stromes beträgt 99°C; das Rücklaufverhältnis 0,2. Über die erste Verbindungsleitung (9) werden 240 kg/h eines TEA reichen Stromes gasförmig entnommen und in die zweite Kolonne (8) eingeleitet.

Die mit einer Gewebepackung ausgestattete Kolonne (8) wird bei 3,5 mbar Kopfvakuum betrieben. Das eintretende Zulaufgemisch besitzt eine Temperatur von 183°C. Das Rücklaufverhältnis der Kolonne beträgt 0,4. Über die zweite Verbindungsleitung (10) werden stündlich 70 kg eines Stromes zurück in die erste Kolonne (1) geleitet. Der 179°C heiße Strom enthält 98,9 % TEA, 0,4°C DEA und 0,7 % Ethanolamin-Ether. Am Kopf (11) dieser Kolonne fallen bei einer Temperatur von 171°C 170 kg/h TEA mit einer Reinheit von 99,6 % an. Die Farbzahl dieses Produktes beträgt 2 APHA.

### Beispiel 2

2300 kg eines Gemisches aus 1800 kg TEA, 490 kg DEA; 20 kg Ethanolamin-Ether, 500 ppm Wasser und 500 ppm Phosphorige Säure werden stündlich über die Zulaufleitung (17) einer Trennwandkolonne (12) gemäß Fig. 2 zugeführt. Die Kolonne wird bei 3 mbar Kopfvakuum betrieben. Die Zulauftemperatur beträgt ca. 100°C. Über den Kopfabzug (18) werden ca. 2400 kg/h eines DEA reichen Stromes entnommen. Etwa 800 kg/h davon werden in die DEA-Aufarbeitung zurückgeführt. Die restlichen 1600 kg/h gelangen als Rücklauf wieder in die Trennwandkolonne zurück, wobei die Menge hälftig auf den Zulaufteil (14) und den Entnahmeteil (15) verteilt wird. Etwa 450 kg/h Schwersieder werden im Sumpf der Kolonne angereichert und mit einer Temperatur von 190°C über den Sumpfabzug (19) ausgeschleust. Im Entnahmeteil (15) der Trennwandkolonne (12) wird TEA aufkonzentriert und mit einer Rate von 1050 kg/h flüssig über den Seitenabzug (20) entnommen. Es wird TEA mit einem Reinheitsgrad von 99,5 % erhalten. Der DEA-Gehalt beträgt 0,1%.

## Patentansprüche

1. Verfahren zur kontinuierlichen, destillativen Abtrennung von Triethanolamin aus einem durch die Umsetzung von Ammoniak mit Ethylenoxid in flüssiger Phase unter Druck und bei erhöhter Temperatur erhaltenen Stoffgemisch aus Mono-, Di- und Triethanolamin sowie Ethanolamin-Ether und Wasser, **dadurch gekennzeichnet, dass** das Stoffgemisch in zwei Stufen destilliert wird, wobei in der ersten Stufe die Leichtsiederfraktion und die Schwersiederfraktion entnommen und ausgeschleust werden und in der zweiten Stufe die Mittelsiederfraktion mit einem Gehalt an Triethanolamin von > 99,4 Gew.-% und Diethanolamin von < 0,2 Gew.-% destilliert wird.

2. Verfahren nach Anspruch 1, **dadurch gekennzeichnet, dass** die Destillation des Stoffgemischs in einer ersten Kolonne und einer mit dieser zusammengeschalteten zweiten Kolonne erfolgt.

3. Verfahren nach Anspruch 1, **dadurch gekennzeichnet, dass** die Destillation des Stoffgemischs in einer Trennwandkolonne erfolgt.

4. Verfahren nach Anspruch 3, **dadurch gekennzeichnet, dass** im mittleren Bereich der Trennwand das Stoffgemisch in die Kolonne zugeführt und Triethanolamin aus der Kolonne ausgetragen wird.

## Claims

1. A process for separating triethanolamine continuously by distillation from a mixture of monoethanolamine, diethanolamine and triethanolamine together with ethanolamine ethers and water obtained by reaction of ammonia with ethylene oxide in the liquid phase under superatmospheric pressure and at elevated temperature, which comprises distilling the mixture in two stages, where the low-boiling fraction and the high-boiling fraction are taken off and discharged in the first stage and the intermediate-boiling fraction comprising > 99.4% by weight of triethanolamine and < 0.2% by weight of diethanolamine is distilled in the second stage.

2. The process according to claim 1, wherein the distillation of the mixture is carried out in a first column and a second column connected to this.

3. The process according to claim 1, wherein the distillation of the mixture is carried out in a dividing wall column.

4. The process according to claim 3, wherein the mixture is fed into the column in the middle region of the dividing wall and triethanolamine is discharged from the column.

## Revendications

1. Procédé de séparation par distillation en continu de triéthanolamine à partir d'un mélange de substances, constitué de monoéthanolamine, de diéthanolamine et de triéthanolamine, ainsi que d'éther d'éthanolamine et d'eau, obtenu par la réaction d'ammoniac avec de l'oxyde d'éthylène en phase liquide sous pression et à température plus élevée, **caractérisé en ce que** le mélange de substances est distillé en deux étapes, dans lequel la fraction à bas point d'ébullition et la fraction à point d'ébullition élevé sont prélevées et évacuées dans la première étape et la fraction à point d'ébullition moyen ayant une teneur en triéthanolamine > 99,4 % en poids et en diéthanolamine < 0,2 % en poids est distillée dans la deuxième étape.

2. Procédé selon la revendication 1, **caractérisé en ce que** la distillation du mélange de substances a lieu dans une première colonne et une deuxième colonne interconnectée avec celle-ci.

3. Procédé selon la revendication 1, **caractérisé en ce que** la distillation du mélange de substances a lieu dans une colonne à paroi de séparation.

4. Procédé selon la revendication 3, **caractérisé en ce que** le mélange de substances est amené dans la colonne au niveau du secteur médian de la paroi de séparation et la triéthanolamine est retirée de la colonne.
